# EUROPEAN PATENT APPLICATION

(11) **EP 1 941 910 A1**
(43) Date of publication of application: **09.07.2008**
(21) Application number: 06769363.0
(22) Date of filing: 06.06.2006
(51) Int. Cl.: A61K 45/06, A61P 19/02

(54) **PHARMACEUTICAL FORM FOR THE TREATMENT OF PATIENTS WITH ACUTE CASES AND EXACERBATIONS OF RHEUMATOID ARTHRITIS AND RELATED ACUTE DISORDERS**

(30) Priority: 29.09.2005 MX PA05010506
(71) Applicant: ESPINOZA ABDALA, Leopoldo de Jesús, Jalisco Mexico (MX); WORLD-TRADE IMPORT-EXPORT, WTIE, A.G., 6302 Zug (CH)
(72) Inventor: GARCÍA ARMENTA, María Elena, C.P. 45020 Guadalajara Jalisco (MX); ÁLVAREZ OCHOA, Víctor Guillermo, C.P.45222 Zapopan Jalisco (MX); SANTOS MURILLO, Josefina, C.P. 44600 Zapopan Jalisco (MX); FLORES BARBA, Sandra Cecilia, C.P. 45170 Zapopan Jalisco (MX); ÁLVAREZ ÁLVAREZ, Aracely, C.P. 45235 Tlaquepaque Jalisco (MX)
(74) Representative: Carvajal y Urquijo, Isabel
(86) International application number: PCT/MX2006/000049
(87) International publication number: WO 2007/037667

(57) **Abstract**

The invention relates to the pharmaceutical industry in general and to the pharmaceutical industry involved in the production of different pharmaceutical formulations containing non-steroidal anti-inflammatories and muscle relaxants and having a central analgesic action. More specifically, the invention relates to novel pharmaceutical formulations containing: (a) non-steroidal anti-inflammatories, (b) muscle relaxants, (c) analgesics, (d) one or more anti-adherent agents, (e) one or more disintegrating agents, (f) one or more binding agents, (g) one or more lubricants, (h) one or more diluents, (i) one or more flavouring agents, (j) one or more surfactants, (k) one or more wetting agents, (1) one or more suspension agents, (m) one or more sweetening agents, (n) one or more flocculating agents, (o) one or more preservatives, (p) one or more antimicrobial agents, (q) one or more pH-buffering systems and (r) one or more solvents and/or excipients, (s) one or more toning agents, (t) one or more antioxidants, (u) one or more chelating agents and any other additive necessary for the formulation.

## Description

### FIELD OF THE INVENTION

The present invention is related generally to the pharmaceutical industry, and particularly to the pharmaceutical industry that produces various pharmaceutical formulations containing non-steroidal anti-inflammatory agents, muscle relaxants of central action and analgesics.

### BACKGROUND OF THE INVENTION

Solid pharmaceutical forms are preparations that contain the active principle(s) and additives, generally disc-shaped, scored or non-scored, and of varying size obtained by compressing powders or granules into capsules, patches, pessaries, beads, suppositories, troches or lozenges.

A suspension is a biphasic system consisting in a finely divided solid dispersed in a solid, a liquid or a gas.

An emulsion is a heterogeneous system comprised of two liquids non-miscible to each other, wherein the dispersed phase is comprised of small droplets distributed in the vehicle in which they are immiscible. The active principle(s) can be in the external or in the internal phase.

A solution is a clear and homogeneous liquid preparation obtained by dissolving the active principle(s) and additives in water, and which is employed for external or internal use. In case of injectable, eye or ear solutions, they should be sterile solutions.

A semi-solid is a preparation that up to certain point is a solid having a stiffness and a viscosity between that of a solid and a liquid.

A syrup is an aqueous solution with a high content of carbohydrates such as: sucrose, sorbitol, dextrose, etc, of viscous consistency in which the active principle(s) and the additives are dissolved.

Analgesics are active principles that relief pain by raising the pain threshold, without altering awareness or others sensorial functions. Certain analgesics can also have anti-rheumatic and anti-inflammatory properties that are used for treatment of arthritis and other inflammation process. A mechanism by means of which some analgesics eliminate pain is based on the finding of opiod receptors in certain parts of the central nervous system and the subsequent identification of a brain's endogenous substance having similar properties to those of morphine. Several of these active principles that fall in this classification, opioids, are regulated substances due to the high risk of this substances to create addiction.

Many drugs used to relief pain are not analgesics. Systemic anesthetics eliminate pain by interfering in the awareness state; local anesthetics prevent pain by blocking peripheral fibers, and supra-renal corticosteroids relief pain associated to rheumatoid arthritis by virtue of its anti-inflammatory action.

Analgesic, antipyretic and anti-inflammatory drugs constitute a small and heterogeneous group of compounds that does not have significant ability to produce addiction and therefore they are not under regulation. Many of these drugs act on fever, pain and inflammation and they are designated as non-steroidal anti-inflammatory drugs (NSAIDs), they are used widely for pain and minor discomfort, headaches and general malaise accompanying feverish illnesses, and to alleviate symptoms from rheumatic fever, arthritis, gout and other musculoskeletal alterations. Among NSAID's there is a sub-division which indicates that these compounds can be classified as non-salicylate NSAID's and salicylate NSAID's.

Among these drugs or active principles we can find those mentioned in Table 1:

**TABLE 1**

| NSAID | |
|---|---|
| Non-salicylate | Salicylate |
| Fenoprofen | Aspirin |
| Flurbiprofen | Choline salicylate |
| Ibuprofen | Magnesium salicylate |
| Ketoprofen | Paracetamol |
| Naproxen | Salicylamide |
| Diclofenac | Salsalate |
| Etodolac | Sodium thiosalicylate |
| Indometacin | |
| Ketorolac | |
| Sulindac | |
| Tolmetin | |
| Meclofenamate | |
| Mefenamic acid | |
| Piroxicam | |
| Meloxicam | |
| Nabumetone | |

The following are some chemical structures of NSAID's:

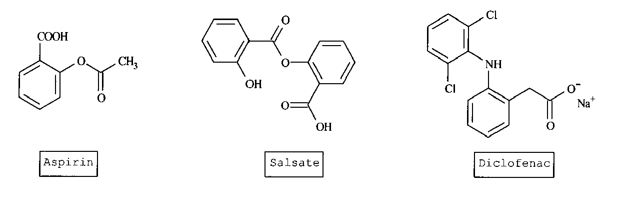

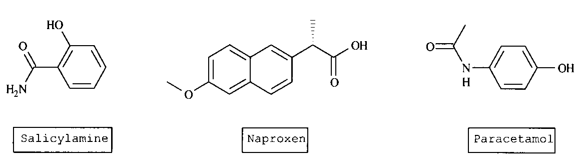

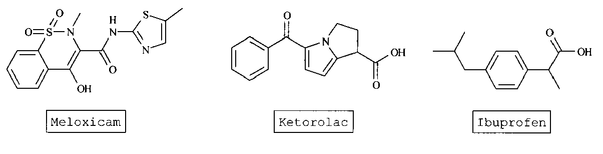

Among drugs with anti-inflammatory activity we can also find steroids. Clinical experience points out that the anti-inflammatory activity of steroids from suprarenal cortex in the human being is well correlated to its glucocorticoid activity. It has been shown that there are more than 50 steroids, but only seven of them exert a proven significant biological effect related to the glucocorticoid and mineralocorticoid function. All suprarenal corticoids, with the exception of androgens, contain 21 carbon atoms, an α,β-insaturated ketone in ring A and an α-ketol chain (-COCH₂OH) attached to ring D, the difference being the extent of oxygenation or hydroxylation in carbons 11, 17 or 19.

Glucocorticoids exert a regulatory influence on lymphocytes, erythrocytes, and eosinophils from blood and on the structure and function of lymphoid tissue, carbohydrates and protein metabolism. Currently, synthetic steroids having greater glucocorticoid activity have been prepared and marketed.

Clinical interest on glucocorticoids is mainly focused, as mentioned above, in their anti-inflammatory effects (by preventing the release of lytic enzymes which not only extend the tissue damage during inflammation but also generate leukotactic substances, decrease phagocytosis and delay migrations of polymorphonuclear leucocytes) and immunosuppressive substances (by effect of phagocytosis suppression, immuno-information processing and by a reduction in the number of eosinophils and lymphocytes).

All corticosteroids are rapidly and completely absorbed in the gastrointestinal tract; a good absorption through skin is also obtained. Some of them are rapidly destroyed by means of first step effect and therefore they should be administered parenterally in order to obtain systemic effects. Many glucocorticoids are also metabolized in skin. In plasma, corticosteroids bind to corticoid-binding protein and albumin, which act as carrier vehicles. Corticosteroids cross the placenta and they can cause congenital malformations. They are also secreted in maternal milk causing suppression of the infant's growth.

The most known application of the anti-inflammatory actions of glucocorticoids is likely to be associated with treatment of arthritic and rheumatic disorders.

The main corticosteroids with anti-inflammatory action are listed in Table 2.

**TABLE 2**

| |
|---|
| Beclometasone dipropionate* |
| Betametasone* |
| Cortisone acetate* |
| Dexametasone* |
| Fludrocostisone acetate* |
| Flunisolide* |
| Fluocinnolone acetonide |
| Fluocinnonide* |
| Flurandrenolide* |
| Hydrocortisone* |
| Methylprednisolone* |
| Prednisolone* |
| Prednisone* |
| Triamcinolone* |
| * and their biologically active salts |

Some of the chemical structures of corticosteroids are shown below:

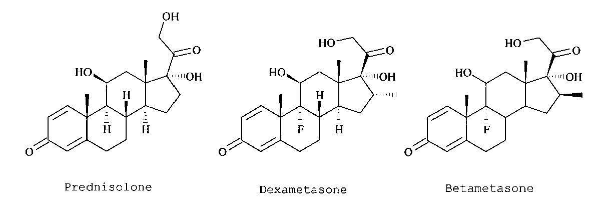

As part of our method for treatment of acute cases and exacerbations of patients suffering from rheumatoid arthritis, ankylosing spondylitis, osteoarthritis, relief of pain and inflammation from acute goutous arthritis, acute and sub-acute bursitis, tendinytis and synovitis, the use of central action muscle relaxants which exert their effect in spinal chord has been considered, where drugs inhibit the mono-synaptic and polysynaptic reflexes. For the above, they have sedative effects as result of the reticulospinal depression, as well as antispasmodic effects of skeletal muscle caused by traumatisms, inflammation osteoarthritis, rheumatoid arthritis, and vertebral disk syndrome. This kind of drugs inhibit polysynaptic reflexes in spinal chord and subcortical regions of the brain, reduce the tone and muscular stress relieving pain and spasms associated with musculoskeletal disorders such as fibrositis, bursitis, spondilitis and muscular injuries.

Among this drugs or active principles we can find those mentioned, among others, in Table 3:

**TABLE 3**

| |
|---|
| Baclofen |
| Carisoprodol |
| Cyclobenzaprine hydrochloride |
| Chlorphenesin carbamate |
| Methocarbamol |
| Orphenadrine citrate |

And some of their chemical structures are as well illustrated below:

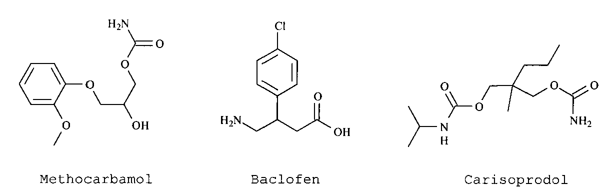

### SUMMARY OF THE INVENTION

The present invention refers to formulations in different pharmaceutical forms containing active principles, which exerts a non-steroidal anti-inflammatory action, muscle relaxants of central action and analgesics.

Specifically, it provides novel formulations in different pharmaceutical forms comprising non-steroidal anti-inflammatory agents, muscle relaxants and analgesics, which achieve a synergistic effect in its function with said combination.

To complement the pharmaceutical form, the composition can contain in addition one or more of the following additives: (a) one or more anti-adherent agents, (b) one or more disintegrating agents, (c) one or more binding agents, (d) one or more lubricating agents, (e) one or more diluting agents, (f) one or more flavoring and/or perfuming agents, (g) one or more surface active agents, (h) one or more wetting agents, (i) one or more suspending agents, (j) one or more sweetening agents, (k) one or more flocculating agents, (1) one or more preserving agents, (m) one or more antimicrobial agents, (n) one or more pH buffering agents, (o) one or more solvents and/or vehicles, (p) one or more tonicity agents, (q) one or more antioxidants, (r) one or more chelating and/or complexing agents, (s) and any other additive required for the formulation.

In other aspect, the present invention also provides a method for treatment of acute cases and exacerbations of patients suffering from rheumatoid arthritis, ankylosing spondylitis, osteoarthritis, pain and inflammation relief from acute gouty arthritis, acute and subacute bursitis, tendonitis, and synovitis.

Drug concentrations in the formulation are in an amount from 0.001% to 100.0%

### DETAILED DESCRIPTION OF THE INVENTION

The present invention refers to formulations in different pharmaceutical forms containing active principles which exerts a non-steroidal anti-inflammatory action, muscle relaxants of central action and analgesics.

Specifically, it provides novel pharmaceutical formulations comprising non-steroidal anti-inflammatory agents, muscle relaxants and analgesics, which achieve a synergistic effect in its function with said combination.

To make a pharmaceutical form, a series of ingredients can be employed such as: anti-adherents such as: talc, colloidal silicon dioxide, calcium sulfate, calcium chloride, among others, with colloidal silicon dioxide being preferred. The anti-adhering agent should be contained in an amount varying from 0.01% to 10.0% with respect to total weight of the formulation.

Disintegrants such as corn starch, modified starches, water soluble cellulose derivatives, sodium crosscarmelose, sodium starch glycolate, binders such as gelatin, natural gums, cellulose derivatives, corn starch, rice starch, polyvinylpirrolidone, povidone, calcium alginate, polyethylene glycols, corn syrup, sucrose, among others, with sodium crosscarmelose being preferred. The disintegrating agent should be contained in an amount varying from 0.25% to 20.0% based on the total weight of the formulation.

Lubricants such as stearic acid, magnesium stearate, talc, among others, with magnesium stearate being preferred. The lubricating agent should be contained in an amount varying from 0.01% to 10.0% with respect to the total weight of the formulation.

Diluents such as microcrystalline cellulose, lactoses, dextrose, sucrose, fructose, phosphates, among others, with sucrose being preferred. The diluting agent should be contained in an amount varying from 0.01% to 90.0% with respect to the formulation total weight.

Surfactants such as poloxamers, sodium lauryl sulfate, sodium docusate, lecithin, polyoxyethylene (20) sorbitan monooleate, among others, with the poloxamers being preferred. The surfactant agent should be contained in an amount varying from 0.01% to 20.0% with respect to the formulation total weight.

The wetting agents can be polar and non-polar, such as ethyl alcohol, water, propylene glycol, polyethylene glycol, glycerol, dimethylacetamide, lecithin, PEG-40 castor oil, butylene glycol, among others, preferably alcohols of 2 carbons such as ethyl alcohol are employed contained in an amount from 1.0% to 50.0% with respect to the total weight of the formulation, excluding the alcohol, and with water being preferred as the polar solvent, contained in an amount varying from 0.01% to 90.0% with respect to the total weight of the formulation.

Flavoring and/or perfuming agents either in powder or liquid form, such as vanilla, cherry, apple, grape, banana, strawberry, peppermint, pineapple, raspberry, chocolate, coconut, peach, lemon, gooseberry, lime, orange, tangerine, among others, synthetic or naturally occurring, with cherry and grape flavors being preferred. The flavoring agent(s) should be contained in an amount from 0.10% to 15% with respect to the total weight of the formulation.

Sweetener(s) such as aspartame, acesulfame K, monosodium glutamate, saccharine, sodium saccharine, among others, with acesulfame K and aspartame being preferred. The sweetener agent should be contained in an amount from 0.00001% to 5.0% with respect to the total weight of the formulation.

Viscosifying agent such as guar gum, acacia gum, sodium carboxymethylcellulose, calcium carboxymethyl cellulose, bentonite, carbomers, carragenine, microcrystalline cellulose and sodium carboxymethyl cellulose, dextrines, hydroxypropylmethyl cellulose, hydroxypropyl cellulose, tragacanth gum, xanthan gum, povidone, pectine, methylcellulose, among others, with sodium carboxymethylcellulose being preferred. The viscosifying agent should be in an amount from 0.01% to 20.0% with respect to the total weight of the formulation.

pH buffering system such as phosphates, citrates, carbonates, bicarbonates, acetates, lactates, among others, with phosphates being preferred. The pH buffering system should be contained in an amount from 0.01% to 10% of the formulation total weight.

Flocculating agent such as sodium chloride, potassium chloride, trisodium citrate, among others, with sodium chloride being preferred. The flocculating agent should be in an amount from 0.0001% to 10% of the total weight of the formulation.

Antimicrobials such as parabens, thymerosal, sodium benzoate, sorbic acid, potassium sorbate, benzyl alcohol and all the antimicrobials that could be used for these pharmaceutical forms. The antimicrobial should be in an amount from 0.0001% to 5% of the formulation total weight.

Solvents and/or vehicles such as ethyl alcohol, corn, sesame, mineral, cottonseed, almond, peanut and soybean oils, glycerin, isopropyl alcohol, polyethylene glycol, propylene glycol, water, sorbitol, ethyl oleate and all solvents and/or vehicles that could be used for these pharmaceutical forms. The solvent and/or vehicle should be in an amount from 0.0001% to 99% of the formulation total weight.

Tonicity agents such as dextrose, glycerin, mannitol, sodium and potassium chloride and all the tonicity agents that could be used for these pharmaceutical forms. The tonicity agent should be in an amount from 0.0001% to 50% of the formulation total weight.

Antioxidant agents such as ascorbic acid, ascorbyl palmitate, butylated hydroxytoluene, butylated hydroxyanisol, sodium metabisulfite, tocopherols, sodium thiosulfate and all the antioxidant agents that could be used for these pharmaceutical forms. The antioxidant agent should be in an amount from 0.001% to 5.0% of the formulation total weight.

Chelating and/or complexing agents such as disodium edetate, edetic acid, cyclodextrines and all the chelating and/or complexing agents that could be used for these pharmaceutical forms. The chelating and/or complexing agent should be in an amount from 0.001% to 10.0% of the formulation total weight.

Sequestering agents such as cyclodextrines and all the sequestering agents that could be used for these pharmaceutical forms. The sequestering agent should be in an amount from 0.001% to 20.0% of the formulation total weight.

Optionally, the formulation can be coated using as a coating material the methacrylates, cellulose derivatives, a blend of polymers and polysaccharides for coating by film formation with aqueous or organic solvents, with coloring agents, flavoring agents, sugars and any other ingredient that is used for film forming, coating and dragee making applications, preferably in an sufficient amount to obtain the desired protection of the pharmaceutical form. Optionally, the formulation can also be scored or non-scored. Optionally, the formulation can be filled in hard or soft gelatin capsules.

The formulation, optionally, can contain in addition other components such as stabilizing agents like creatinine or dextrines, adsorbents such as bentonite, magnesium carbonate, powdered cellulose, colorants and/or pigments such as caramel, ferric oxide, red, yellow, black, mixture thereof, and all the colorants and/or pigments that could be used for these pharmaceutical forms.

The formulations can be contained in containers with suitable capacity which can include a blisterpack made of P.V.C. (polyvinyl chloride) film with a thickness from 200 µ to 250 µ, which optionally can have a coating of PVDC (polyvinylidene chloride) with a weight from 25 g/m² to 120 g/m², and bonded with aluminum foil or in cellopolyal film and also in vials of suitable capacity elaborated from high and/or low density polyethylene, polyethylene terephthalate, polyvinyl chloride, polypropylene, polystyrene, Type I, II, III and IV glass, and any other primary packaging material, with or without color. The cap may be of the following types: tamperproof, threaded, cap-to-cap, child proof, elaborated from high and/or low density polyethylene, polyethylene terephthalate, polyvinyl chloride, polypropylene, polystyrene, with or without color. Dosing auxiliaries, such as dosing spoons, metering cups, pipettes, small syringes, inserts elaborated from materials such as, *inter alia*, high and/or low density polyethylene, polyethylene terephthalate, polyvinyl chloride, polypropylene, polystyrene, with or without color, are also included.

The elaboration is carried out mixing all the components required for the formulation.

For treatment of acute cases and exacerbations of patients suffering from rheumatoid arthritis, ankylosing spondylitis, osteoarthritis, pain and inflammation relief from acute gouty arthritis, acute and subacute bursitis, tendonitis, synovitis.

Therefore, the present invention provides a pharmaceutical formulation, which provides one or more non-steroidal anti-inflammatory agent, one or more muscle relaxants and one or more analgesics.

### EXAMPLES

The following non-limiting examples will assist to illustrate the present invention:

### EXAMPLE 1

The amounts are in percent wt/wt

| | | |
|---|---|---|
| 1) | Methocarbamol | 40.0000 |
| 2) | Meloxicam | 0.0500 |
| 3) | Bethamethasone | 0.0050 |
| 4) | Sucrose for compression | 50.0000 |
| 5) | Polyvidone k-30 | 5.0000 |
| 6) | 96% Ethyl alcohol | 20.0000 |
| 7) | Talc | 5.0000 |
| 8) | Purified water | 4.0000 |

The preparation of capsules is carried out as follows: A solution is prepared mixing the solvents, purified water and 96% ethyl alcohol, to this solution Povidone is added and dissolved therein. Next, the active principles Methocarbamol, Meloxicam and Bethamethasone are mixed and then wetted with the solution that contains Povidone. The resulting mixture is dried, screened and thereafter sucrose for compression and talc are added. Proceed to encapsulation.

### EXAMPLE 2

The values are in % wt/vol

| | | |
|---|---|---|
| 1) | Methocarbamol | 30.000 |
| 2) | Meloxicam | 0.500 |
| 3) | Bethamethasone | 0.050 |
| 4) | Sodium carboxymethyl cellulose | 10.000 |
| 5) | Colloidal silicon dioxide | 1.000 |
| 6) | 96% ethyl alcohol | 2.000 |
| 7) | Dextrose | 70.000 |
| 8) | Flavor | 0.500 |
| 9) | Color | 0.010 |
| 10) | Propylene glycol | 20.000 |
| 11) | Sodium benzoate | 0.010 |
| 12) | Purified water, q.s. | 100.000 |

The elaboration of the suspension is carried out as follows: carboximethyl cellulose, dextrose, sodium benzoate, coloring agent and flavoring agent are dissolved in a portion of water. Then, 96% ethyl alcohol, propylene glycol and colloidal silicon dioxide are added, this is stirred to homogenize the suspension, then Bethamethasone, Meloxicam and Methocarbamol are added and the mixture is stirred until a homogeneous suspension is obtained. Finally water is added to the desired volume.

### EXAMPLE 3

The values are in percent wt/wt

| | | |
|---|---|---|
| 1) | Methocarbamol | 0.20 |
| 2) | Meloxicam | 0.05 |
| 3) | Bethamethasone | 0.50 |
| 4) | Carbomer | 10.00 |
| 5) | Sodium hydroxide | 5.00 |
| 6) | 96% ethyl alcohol | 4.00 |
| 7) | Propyl parabene | 2.00 |
| 8) | Methyl parabene | 0.01 |
| 12) | Purified water, q.s. | 80.00 |

The elaboration of in an amount of purified water the carbomer is dispersed, once it is dispersed, in a separate vessel the sodium hydroxide is dissolved and then this is added slowly and with continuous stirring to the carbomer dispersion. The 96% ethyl alcohol is added to another amount of purified water and the propyl parabene, methyl parabene, bethamethasone, meloxicam and methocarbamol are dissolved in this solution. Add the active-containing solution and this is added to the matrix and stirred until homogenization.

The invention has been described sufficiently as to allow that a person of ordinary skill in the art could realize and obtain the results mentioned in the present invention. However, any person of ordinary skill in the art to which the present invention pertains could be able to make modifications not disclosed in the present application, nonetheless, if for the application of these modifications in a given structure or in the manufacturing process thereof, the subject matter claimed in the following claims is required, said structures should be embraced within the scope of the invention.

## Claims

1. Pharmaceutical forms comprising: (a) non-steroidal anti-inflammatories, (b) muscle relaxants, (c) analgesics, (d) one or more anti-adherent agents, (e) one or more disintegrating agents, (f) one or more binding agents, (g) one or more lubricating agents, (h) one or more diluting agents, (i) one or more flavoring agents, (j) one or more surface active agents, (k) one or more wetting agents, (1) one or more suspending agents, (m) one or more sweetening agents, (n) one or more flocculating agents, (o) one or more preserving agents, (p) one or more antimicrobial agents, (q) one or more pH buffering systems, and (r) one or more solvents and/or vehicles, (s) one or more tonicity agents, (t) one or more antioxidants, (u) one or more chelating agents, and any other additive required for the formulation.

2. A formulation as claimed in claim 1, wherein the concentrations of the active principles in the formulation are in an amount from 0.001% to 100.0%.

3. A formulation as claimed in claim 2, wherein the combined active principles therein can be found as it anhydrous, monohydrated or dehydrated base or as a physiologically acceptable salt.

4. A formulation as claimed in claim 3, wherein it contains one or more of the following ingredients: anti-adherents such as: talc, colloidal silicon dioxide, calcium sulfate, calcium chloride, among others. Disintegrants such as corn starch, modified starches, water soluble cellulose derivatives, sodium crosscarmelose, sodium starch glycolate, binders such as gelatin, natural gums, cellulose derivatives, corn starch, rice starch, polyvinylpirrolidone, povidone, calcium alginate, polyethylene glycols, corn syrup, sucrose, among others. Lubricants such as stearic acid, magnesium stearate, talc, among others. Diluents such as microcrystalline cellulose, lactoses, compressible sugar, dextrose, sucrose, fructose, among others. Surfactants such as poloxamers, sodium lauryl sulfate, sodium docusate, lecithin. Polar and non-polar wetting agents, such as ethyl alcohol, water, propylene glycol, polyethylene glycol, glycerol, dimethylacetamide, lecithin, PEG-40 castor oil, butylene glycol, among others. Flavoring and/or perfuming agents either in powder or liquid form, such as vanilla, cherry, apple, banana, strawberry, peppermint, pineapple, raspberry, chocolate, coconut, peach, lemon, gooseberry, lime, orange, tangerine, among others, synthetic or naturally occurring. Sweetener(s) such as aspartame, acesulfame K, monosodium glutamate, saccharine, sodium saccharine, among others. Viscosifying agent such as guar gum, acacia gum, sodium carboxymethylcellulose, calcium carboxymethyl cellulose, bentonite, carbomers, carragenine, microcrystalline cellulose and sodium carboxymethyl cellulose, dextrines, hydroxypropylmethyl cellulose, hydroxypropyl cellulose, tragacanth gum, xanthan gum, povidone, pectin, methylcellulose, among others. pH buffering system such as phosphates, citrates, carbonates, bicarbonates, acetates, lactates, among others. Flocculating agent such as sodium chloride, potassium chloride, trisodium citrate, among others. Antioxidant agents such as ascorbic acid, ascorbyl palmitate, butylated hydroxytoluene, butilated hydroxyanisol, sodium metabisulfite, tocopherols, sodium thiosulfate among others. Tonicity agents such as dextrose, glycerin, mannitol, sodium and potassium chloride among others. Solvents and/or vehicles such as ethyl alcohol, corn, sesame, mineral, cottonseed, almond, peanut and soybean oils, glycerin, isopropyl alcohol, polyethylene glycol, propylene glycol, water, sorbitol, ethyl oleate among others. Chelating and/or complexing agents such as disodium edetate (EDTA), ascorbic acid, Butyl hydroxytoluene (BHT), tocopherols, sodium bisulfite, sodium metabisulfite among others. Antimicrobials such as parabenes, thymerosal, sodium benzoate, sorbic acid, potassium sorbate, benzyl alcohol and all the antimicrobials that could be used for these pharmaceutical forms.

5. A formulation as claimed in all the preceding claims, wherein said formulation can be a dragee, tablet, coated tablet, capsule, suspension, syrup, emulsion, troches or lozenges, or solution, with capsules being preferred.

6. The formulation that might contain a coating agent, that uses as a coating material the methacrylates, cellulose derivatives, a blend of polymers and polysaccharides for coating by film formation with aqueous or organic solvents, which also has coloring agents, flavoring agents, sugars and any other ingredient that is used for film forming, coating and dragee making applications.

7. A formulation that might be encapsulated in hard or soft gelatin capsules. The formulation that might also contain other components such as stabilizing agents like creatinine or dextrines, among others. Furthermore, adsorbents such as bentonite, magnesium carbonate, powdered cellulose,among other may also be included.

8. A formulation as claimed in claim 7, wherein said formulation is contained in containers with suitable capacity which can include a blister pack made of PVC (polyvinyl chloride) film with a thickness from 200 µ to 250 µ, which optionally can have a coating of PVDC (polyvinylidene chloride) with a weight from 25 g/m² to 120 g/m², and bonded with aluminum foil or in cellopolyal film and also in bottles of suitable capacity elaborated from high and/or low density polyethylene, polyethylene terephthalate, polyvinyl chloride, polypropylene, polystyrene, Type I, II, III and IV glass, and any other primary packaging material, with or without color. The cap may be of the following types: tamperproof, threaded, cap-to-cap, child proof, elaborated from high and/or low density polyethylene, polyethylene terephthalate, polyvinyl chloride, polypropylene, polystyrene, with or without color. Dosing auxiliaries, such as dosing spoons, metering cups, pipettes, small syringes, inserts elaborated from materials such as, *inter alia,* high and/or low density polyethylene, polyethylene terephthalate, polyvinyl chloride, polypropylene, polystyrene, among others, with or without color, are also included.

9. A method for treatment of acute cases and exacerbations of patients suffering from rheumatoid arthritis, ankylosing spondylitis, osteoarthritis, pain and inflammation relief from acute gouty arthritis, acute and subacute bursitis, tendonitis, synovitis, by administering of one or more non-esteroidal anti-inflammatory agents, one or more muscle relaxants, and one or more analgesics.

10. A solid pharmaceutical formulation which provides therapeutically suitable doses of one or more non-esteroidal anti-inflammatory agents, one or more muscle relaxants, and one or more analgesics.
